# EUROPEAN PATENT SPECIFICATION

(11) **EP 4 518 873 B1**
(45) Date of publication and mention of the grant of the patent: **03.12.2025**
(21) Application number: 23732186.4
(22) Date of filing: 03.05.2023
(51) Int. Cl.: A61K 33/00, A23L 33/16, A61K 9/00, A61K 31/122, A61K 31/593, A61P 19/10

(54) **COMPOSITION COMPRISING BIOAVAILABLE SILICON AND VITAMINS**
ZUSAMMENSETZUNG MIT BIOVERFÜGBAREM SILICIUM UND VITAMINEN
COMPOSITION À BASE DE SILICIUM BIODISPONIBLE ET DE VITAMINES

(30) Priority: 04.05.2022 IT 202200009089
(43) Date of publication of application: 12.03.2025
(73) Proprietor: Geofarma S.r.l., 70042 Mola di Bari (BA) (IT)
(72) Inventor: LATTARULI, Leonardo, 70042 Mola di Bari (IT); CAMPANILE, Maria Grazia, 70042 Mola di Bari (IT)
(74) Representative: De Tullio, Michele Elio
(86) International application number: PCT/IB2023/054586
(87) International publication number: WO 2023/214321

(56) References cited:
- WO-A1-2019/179611
- US-A1- 2011 293 759
- US-A1- 2022 008 454
- US-B2- 9 132 083
- YONEMURA KATSUHIKO ET AL: "Protective effect of vitamins K2 and D3on prednisolone-induced loss of bone mineral density in the lumbar spine", AMERICAN JOURNAL OF KIDNEY DISEASES, vol. 43, no. 1, January 2004 (2004-01-01), pages 53 - 60, XP028834733, ISSN: 0272-6386, DOI: 10.1053/J.AJKD.2003.09.013
- IWAMOTO J ET AL: "High-dose vitamin K supplementation reduces fracture incidence in postmenopausal women: a review of the literature", NUTRITION RESEARCH, ELSEVIER, AMSTERDAM, NL, vol. 29, no. 4, 1 April 2009 (2009-04-01), pages 221 - 228, XP026089027, ISSN: 0271-5317, [retrieved on 20090502], DOI: 10.1016/J.NUTRES.2009.03.012
- RODELLA LUIGI FABRIZIO ET AL: "A review of the effects of dietary silicon intake on bone homeostasis and regeneration", THE JOURNAL OF NUTRITION, HEALTH, SPRINGER PARIS, PARIS, vol. 18, no. 9, 5 December 2014 (2014-12-05), pages 820 - 826, XP035409703, ISSN: 1279-7707, [retrieved on 20141205], DOI: 10.1007/S12603-014-0555-8
- RAVIN JUGDAOHSINGH ET AL: "Dietary Silicon Intake Is Positively Associated With Bone Mineral Density in Men and Premenopausal Women of the Framingham Offspring Cohort", JOURNAL OF BONE AND MINERAL RESEARCH, vol. 19, no. 2, 16 February 2004 (2004-02-16), US, pages 297 - 307, XP055389888, ISSN: 0884-0431, DOI: 10.1359/JBMR.0301225

## Description

### TECHNICAL FIELD

The present invention relates to a composition comprising bioavailable silicon and vitamins. In particular, the present invention relates to a synergistic composition comprising silicic acid, vitamin D3, and vitamin K2 for the treatment and/or prevention of osteoporosis and bone fragility-related dysfunction.

### BACKGROUND ART

As is well known to experts in the field, silicon is a very important trace element for humans.

Silicon is the fundamental element of proteins responsible for tissue strength and elasticity. Like other important trace elements, for example, copper, iron, cobalt, and zinc, silicon is also incorporated into connective tissues, bones, skin, hair, nails, and blood vessels. In addition, silicon strengthens the body's defense system, the so-called immune system, and promotes wound healing.

Silicon deficiency leads to growth disorders, loss of bone stability with increased risk of osteoporosis and fractures, as well as premature hair loss, brittle nails, and skin changes.

Possible skin changes are increased wrinkling, dryness, flaking, itching, thickening, and painful cracking of the skin due to reduced elasticity. In addition, the immune system is weakened by silicon deficiency, and there is increased susceptibility to infection (US20060178268A1).

As pointed out by a wide scientific literature, the function of silicon as a key trace element in the synthesis of type 1 collagen and osteocalcin, in increasing osteoblast activity, and increasing alkaline phosphatase concentration is well documented, although its mechanism of action has not been fully clarified (Reffitt D. M. et al., Bone 32, 2003, 127-135; Dong M. et al., Biol. Trace Elem. Res. 2016, 173(2), 306-315, and references cited therein). Therefore, silicon has a beneficial effect on mineralization and increased bone density, and, when taken through a balanced diet and/or with the aid of supplements, silicon is a valuable support for the treatment and/or prevention of osteoporosis and dysfunction related to bone fragility, especially in postmenopausal women. The bone fragility-related costs to be borne by the health care systems of Western countries (U.S. and Europe) were estimated to be about $50 billion in 2006, and this value was expected to increase over the next decade (Sambrook et al., 2006, Osteoporosis, Lancet 367: 2010-2018).

In its most naturally occurring form, silicon, the second most abundant element in the Earth's crust, is found in the form of silica SiO2 or a multitude of silicates or aluminosilicates minerals, substances that are poorly soluble and bioavailable to the gastrointestinal tract. The absorbable and bioavailable form is that of silicic acid and its derivatives, such as organosilanolates, and these, therefore, are the substances included in most of the patented liquid and solid formulations currently on the market. In addition, it is well known that silicic acid and its derivatives are very reactive acids, are not stable and, in a strongly acidic pH environment, tend to condense and polymerize into polysilicic acids and hydrated silica, substances that are poorly or not at all bioavailable. Therefore, a stabilizer must be added to the formulation to inhibit this type of polymerization, increasing formulation costs.

Stabilizers can be e.g. phenols and/or polyphenols (as described e.g. in EP2526954A1 and WO2019179611A1), metal chelates from amino acids or aromatic compounds (as described e.g. in WO2018037115A1), fatty acid derivatives (as described e.g. in US2003/0018011A1), polypeptides, polyalcohols ssuch as maltodextrins, quaternary ammonium salts, aldehydes (as described for example in WO2020165415A1 and WO2020094886A1), urea, sorbitol, cellulose derivatives (as described for example in WO2010018418A1), choline (as described for example in WO2003077657). Concurrently, scientific research has demonstrated the importance of K-series vitamins (R. Jugdaohsingh et al., Bone 43, 2008, 596-606; Iwamoto J. et al., Nutr. Res. 2009, 29, 221-228) and D-series vitamins (Cranney A. et al., Evid. Rep. Technol. Assess, 2007, 158, 1-235) as synergistic adjuvants to the beneficial action of silicon on bone. In particular, vitamin K2 acts as a cofactor by promoting osteocalcin activation and contributing to bone mineralization through the binding of the same silicon (Bügel S., Proc. Nutr. Soc. 2003, 62, 839-843; Van Summeren M.J. et al, Br. J. Nutr. 2008, 18, 1-7).

Similarly, vitamin D3 has a beneficial effect on bone collagen and promotes the decrease of calcium and phosphate in blood and soft tissue, promoting their reabsorption at the gastrointestinal level and return to bone mineral tissue (Rabon J.R. et al, Poultry Science, 1995, 74, 352-359; Spector T.D. et al, BMC Musculoskelet. Disord. 9:85).

Surprisingly, there is no evidence of patent documentation on the association of silicic acid with vitamins K2 and D3 in liquid and solid state formulations. The closest known art is document JP2013224269A, which describes bioavailable silicon in association with a riboflavin-based vitamin preparation, having a different function from those of vitamins K2 and D3. Vitamins K2 and D3, by virtue of the presence of oxygen atoms, having considerable affinity for silicon, can contribute to the stabilization of silicon itself without the addition of other chemical stabilizers known in the art. Moreover, formulations currently on the market do not allow potential of said synergistic effect of vitamins K2 and D3 to be exploited for the treatment and/or prevention of osteoporosis and bone fragility-related dysfunction.

Because of these drawbacks, there is therefore still a need for new formulations in the liquid and solid state that involve the combination of silicon in a bioavailable form with vitamins K2 and D3, that do not necessarily involve the use of another stabilizer, and that are capable of the same performance as commercially available formulations. The main purpose of the present invention is to overcome the technical disadvantages mentioned above by combining silicon in bioavailable form with vitamins K2 and D3 for the treatment and/or prevention of osteoporosis and dysfunction related to bone fragility.

### SUMMARY OF INVENTION

This purpose is achieved by the present invention, which concerns a composition comprising:
- a therapeutically effective amount of silicon in a bioavailable form,
- vitamin D3 and vitamin K2,
said components being present in sufficient quantity to provide a synergistic effect. Also said vitamin components being present in sufficient quantity to stabilize said silicon in bioavailable form.

### DETAILED DESCRIPTION OF THE INVENTION

The terms "therapeutically effective amount" in the present description refer to an amount sufficient to achieve, through dosing for a specified period of time, a desired therapeutic effect. A therapeutic effect is not necessarily a cure, and may be, for example, the relieving of symptoms or the general improvement of conditions.

The term "synergistic effect" for a mixture of components, such as those in the present description, is used when the effect achieved, such as a therapeutic effect, is greater than the expected sum of the effects of the components taken individually.

The terms "buffering effect" or "buffering capacity" refer to the ability of a chemical system, such as the composition of this description, to resist a change in pH.

The term "comprising" as used in the present description, before a set of components, features, elements, does not exclude or preclude the presence of other components, features, elements in accordance with techniques known to the expert in the field. The term "bioavailable silicon" in the present description means a preparation containing silicon in the form absorbable by the human body.

The term "IU" (International Unit) referring to vitamin D3 in the present description means an amount such that 1 IU is equivalent to 0.025 µg of said vitamin.

All other terms used in the description of the present invention, unless otherwise specified, shall be interpreted according to their ordinary meaning known to the person skilled in the art.

The present invention in a first aspect relates to a composition comprising:
- a therapeutically effective amount of silicon in a bioavailable form,
- vitamin D3 and vitamin K2,
said vitamin components being present in sufficient quantity to stabilize said silicon in bioavailable form.

Preferably, silicon in bioavailable form is silicic acid, which must be understood as the name for a family of chemical compounds of general formula [SiOₓ(OH)₄₋₂ₓ]ₙ containing the element silicon bonded to the element oxygen and the -OH group. Examples of silicic acid include metasilicic acid H₂SiO₃, linear or cyclic oligomers of formula [SiO(OH)₂]ₙ with n<1000, even more preferably with n<100, orthosilicic acid H₄SiO₄, disilicic acid H₂Si₂O₅, pyrosilicic acid H₄Si₂O₇. More preferably, the silicic acid of the present invention is orthosilicic acid H₄SiO₄.

In a second aspect of the present invention, said composition is a liquid composition for oral use selected from a group comprising aqueous dispersion, O/W emulsion, W/O emulsion, hydrogel, hydrocolloid, micro-encapsulated suspension, nano-encapsulated suspension.

In the preferred embodiment form of the present invention, said composition is an aqueous dispersion with a daily dosage that ranges from 15 mL and 20 mL. The amount of vitamin D3 ranges from 1,0 mg/L and 4,0 mg/L, more preferably from 1,7 mg/L and 3,0 mg/L. The amount of vitamin K2 ranges from 1,0 mg/L and 4,0 mg/L, more preferably from 1,3 mg/L and 3,0 mg/L. The amount of orthosilicic acid used ranges from 2 g/L and 3 g/L, more preferably from 2,2 g/L and 2,4 g/L.

As known to the technical expert, silicic acid is a very reactive acid and in acidic pH environments, such as at pH<2, it tends to polymerize into linear or cyclic structures of general formula [SiO(OH)₂]ₙ, with n>1000, which are poorly or no longer bioavailable. The technical expert will appreciate that, in order to avoid this drawback, the addition of an organic polyprotic acid, selected, for example, from a group comprising citric acid, tartaric acid, maleic acid, or inorganic, such as boric acid, phosphoric acid, in the form of a salt with a metal cation, and mixtures thereof, can have a buffering effect on the composition avoiding a risky lowering of pH and decrease in the bioavailability of silicon. The metal cation is selected from the group comrpising, for example, Li, Na, K, Mg, Ca, Zn, Fe.

In the preferred embodiment form of the present invention, said organic polyprotic acid in salt form is calcium citrate, in amount that ranges from 50 g/L to 200 g/L, preferably from 100 g/L and 150 g/L, in order to stabilize the pH in the range of 3,0 to 4,0. Optionally, calcium citrate can act as a source of bone calcium.

As is known to the technical expert, excipients and additional ingredients are added in liquid dosage forms to complement and stabilize the formulation. Excipients are selected from the group comprising e.g. surfactants, emulsifiers, humectants, dispersants, diluents, binders such as e.g. xylitol, stabilizers such as e.g. gum arabic, rheology modifiers such as e.g. xanthan gum, disintegrants, lubricants, thickeners such as e.g. guar gum, sweeteners such as e.g. steviol glycosides, flavorings, preservatives, antioxidants.

Examples of antioxidants may be tocopherol, tocopherol acetate, or ascorbic acid. Flavorings may be for example mint, blueberry, strawberry, raspberry, lemon, vanilla or mixtures thereof.

Other additional ingredients that may be included in the composition are known in the art and described, for example, in Genaro et al, Remington's Pharmaceutical Sciences, Mack Publishing Co., Easton, Pa., 17th edition.

In order to preserve the quality of the ingredients, mixing of the components is done at a temperature below 25°C, preferably from 10°C and 15°C.

In another aspect of the present invention, said composition is a solid oral form selected from a group comprising tablet, capsule, sachet, granules, and powder, in which the amount of silicon in bioavailable form is equivalent to an amount of elemental silicon preferably from 20% w/w and 30% w/w.

In a preferred form of realization, silicon in bioavailable form is silicic acid, preferably orthosilicic acid in the form absorbable by the human body, in amount that ranges from 10 mg and 300 mg per solid oral form. The amount of vitamin D3 ranges from 2 IU and 2000 IU per solid oral form. The amount of vitamin K2 ranges from 10 µg and 200 µg per solid oral form.

As is known to the technical expert, excipients and additional ingredients are added in the solid dosage forms to complement and stabilize the formulation. Excipients are selected from the group comprising, for example, fillers, binders, disintegrants, lubricants, thickeners, and flavorings. Other additional ingredients that may be included in the composition are known in the art and described, for example, in Genaro et al, Remington's Pharmaceutical Sciences, Mack Publishing Co., Easton, Pa., 17th edition.

Fillers may be selected from the group comprising, for example, microcrystalline cellulose, lactose monohydrate, calcium hydrogen phosphate, sorbitol, starch, modified starches, talc, kaolin, bentonite, montmorillonite, calcium carbonate, magnesium carbonate, calcium silicate, aluminum hydroxide, amorphous silica, or mixtures thereof.

Binders may be selected from the group comprising, for example, gelatin, lactose monohydrate, sorbitol, sucrose, xylitol, maltitol, starch, modified starches, methylcellulose, 2-hydroxyethylcellulose, 2-hydroxypropylcellulose, sodium carboxymethylcellulose, polyethylene glycols, polyglycerols, polyvinylpyrrolidone, polyvinylpyrrolidone copolymers, carrageenans, or mixtures thereof.

Disintegrants may be selected from the group comprising, for example, starch, modified starches, sodium starch glycolate, methylcellulose, sodium carboxymethylcellulose, 2-hydroxyethylcellulose, 2-hydroxypropylcellulose, polyvinylpyrrolidone, polyvinylpyrrolidone copolymers, or mixtures thereof. Lubricants can be selected from the group comprising, for example, magnesium stearate, calcium stearate, zinc stearate, stearic acid, talc, or mixtures of these substances.

Thickeners may be selected from the group comprising, for example, polyacrylic acid, its copolymers, or their sodium, potassium salts, or triethanolamine, methylcellulose, sodium carboxymethylcellulose, 2-hydroxyethylcellulose, 2-hydroxypropylcellulose, maltodextrins, starch, modified starches, rice flour, polyglycerols, polyethylene glycols, gelatin, pectin, agar agar, carrageenans, gum arabic, alginic acid, sodium alginate, or mixtures thereof.

As the person skilled in the art will appreciate, the composition of the present invention can be advantageously employed for the preparation of dietary supplement, beverage or medicament for the treatment and/or prevention of osteoporosis and dysfunction related to bone fragility. The formulation is produced by common processes known to experts in the art of pharmaceutical technology [S. C. Gad et al: Handbook of pharmaceutical manufacturing: production and processes, Wiley (2008)].

In addition, the ecological aspect of industrial production processes is now a major socio-environmental issue. Interest in environmental protection, in addition to ethical implications, also has economical ones: more advanced sectors of consumers are increasingly directing their choices toward products that are more environmentally friendly. In particular, renewability of sources is a highly desired property. The technical expert will appreciate that plant-derived sources such as, for example, field horsetail (Equisetum arvense), pulmonaria (Pulmonaria officinalis), oats (Avena sativa), nettle (Urtica dioica), dandelion root (Taraxacum officinale), and algae can be renewable sources of silicic acid.

Finally, the person skilled in the art will agree that the composition described in the present invention can be adapted through the known art to continuous and discontinuous industrial plants for the preparation of dietary supplement, beverage, or medicament for the treatment and/or prevention of osteoporosis and bone fragility-related dysfunction.

The present invention is illustrated more in detail in a non-limiting manner by the following examples.

### EXAMPLE 1

This example refers to the preparation of one liter of aqueous composition with a recommended daily dosage of 20 mL. They were mixed under stirring at a temperature between 10°C and 15°C:

| | |
|---|---|
| orthosilicic acid: | 2,35 g |
| calcium citrate: | 126 g |
| vitamin D3: | 1,7 to 2,0 mg |
| phosphoric acid: | quantum satis |
| guar gum: | quantum satis |
| xanthan gum: | quantum satis |
| gum arabic: | quantum satis |
| xylitol: | quantum satis |
| steviol glycosides: | quantum satis |
| flavorings: | quantum satis |
| purified water: | quantum satis |

### EXAMPLE 2

This example refers to the preparation of one liter of aqueous composition with a recommended daily dosage of 15 mL. They were mixed under stirring at a temperature between 10°C and 15°C:

| | |
|---|---|
| orthosilicic acid: | 2,35 g |
| calcium citrate: | 126 g |
| vitamin D3: | 1,7 to 2,0 mg |
| vitamin K2: | 1,3 to 3,0 mg |
| phosphoric acid: | quantum satis |
| guar gum: | quantum satis |
| xanthan gum: | quantum satis |
| gum arabic: | quantum satis |
| xylitol: | quantum satis |
| steviol glycosides: | quantum satis |
| flavorings: | quantum satis |
| purified water: | quantum satis |

### EXAMPLE 3

This example refers to the preparation of one liter of aqueous composition with a recommended daily dosage of 15 mL. They were mixed under stirring at a temperature between 10°C and 15°C:

| | |
|---|---|
| orthosilicic acid: | 2,35 g |
| vitamin D3: | 1,7 to 2,0 mg |
| vitamin K2: | 1,3 - 3,0 mg |
| phosphoric acid: | quantum satis |
| guar gum: | quantum satis |
| xanthan gum: | quantum satis |
| gum arabic: | quantum satis |
| xylitol: | quantum satis |
| steviol glycosides: | quantum satis |
| flavorings: | quantum satis |
| purified water: | quantum satis |

### EXAMPLE 4

This example refers to the preparation of one liter of aqueous composition with a recommended daily dosage of 15 mL. They were mixed under stirring at a temperature between 10°C and 15°C:

| | |
|---|---|
| orthosilicic acid: | 2,35 g |
| calcium citrate: | 126 g |
| vitamin D3: | 1,7 to 2,0 mg/L |
| vitamin K2: | 1,3 to 3,0 mg/L |
| phosphoric acid: | quantum satis |
| guar gum: | quantum satis |
| xanthan gum: | quantum satis |
| gum arabic: | quantum satis |
| xylitol: | quantum satis |
| steviol glycosides: | quantum satis |
| flavorings: | quantum satis |
| purified water: | quantum satis |

### EXAMPLE 5

This example refers to the preparation of a typical solid oral form in capsule form:

| | |
|---|---|
| equivalents of orthosilicic acid: | 10 to 300 mg |
| vitamin D3: | 2 IU - 2000 IU |
| vitamin K2: | 10 - 200 µg |
| rice flour: | quantum satis |
| amorphous silica: | quantum satis |

### EXAMPLE 6

This example refers to the preparation of a typical solid oral form in tablet form:

| | |
|---|---|
| equivalents of orthosilicic acid: | 10 to 300 mg |
| vitamin D3: | 2 IU - 2000 IU |
| vitamin K2: | 10 - 200 µg |
| magnesium stearate: | quantum satis |
| amorphous silica: | quantum satis |

### EXAMPLE 7

This example refers to the preparation of a typical solid oral form in the form of a sachet:

| | |
|---|---|
| equivalents of orthosilicic acid: | 10 to 300 mg |
| vitamin D3: | 2 IU - 2000 IU |
| vitamin K2: | 10 - 200 µg |
| maltodextrin: | quantum satis |
| citric acid: | quantum satis |
| sorbitol: | quantum satis |
| amorphous silica: | quantum satis |
| flavorings: | quantum satis |

## Claims

1. A composition comprising a therapeutically effective amount of orthosilicic acid vitamin D3 and vitamin K2, **characterized by** the fact that said composition provides a synergistic effect.

2. The composition according to claim 1, **characterized in that** said composition is a liquid composition for oral use selected from a group comprising aqueous dispersion, O/W emulsion, W/O emulsion, hydrogel, micro-encapsulated suspension, nano-encapsulated suspension.

3. The composition according to claim 2, wherein said composition is an aqueous dispersion.

4. The composition according to claim 3, comprising an organic or inorganic polyprotic acid in the form of a salt with a metal cation, and mixtures thereof, with a buffer function.

5. The composition according to claim 4, wherein said organic polyprotic acid in the form of a salt with a metal cation is calcium citrate ranging from 100 g/L and 150 g/L.

6. The composition according to claims from 2 to 5, wherein the amount of said orthosilicic acid ranges from 2.2 g/L and 2.4 g/L.

7. The composition according to claims from 2 to 5, wherein the amount of vitamin D3 ranges from 1,7 mg/L and 2,0 mg/L and the amount of vitamin K2 ranges from 1,3 mg/L and 3 mg/L.

8. The composition according to claims from 2 to 5, **characterized by** the fact that said composition is administered in a daily dose ranging from 15 mL and 20 mL.

9. The composition according to claim 1, **characterized by** the fact that said composition is a solid oral form selected from a group including tablet, capsule, sachet, granules, powder.

10. The composition according to claim 9, wherein the equivalents of orthosilicic acid range from 10 mg/ solid oral form to 300 mg/ solid oral form.

11. The composition according to claims 9 and 10, wherein the amount of vitamin D3 ranges from 2 IU/ solid oral form to 2000 IU/ solid oral form and the amount of K2 vitamin ranges from 10 µg / solid oral form to 200 µg/ solid oral form.

12. The composition according to anyone of the preceding claims for use in prevention or treatment of osteoporosis and bone fragility-related dysfunction.

## Patentansprüche

1. Zusammensetzung, umfassend eine therapeutisch wirksame Menge an Orthokieselsäure, Vitamin D3 und Vitamin K2,
**dadurch gekennzeichnet, dass** die Zusammensetzung einen synergistischen Effekt bereitstellt.

2. Zusammensetzung nach Anspruch 1, **dadurch gekennzeichnet, dass** die Zusammensetzung eine flüssige Zusammensetzung zur oralen Verwendung ausgewählt aus einer Gruppe ist, die wässrige Dispersion, O/W-Emulsion, W/O-Emulsion, Hydrogel, mikroverkapselte Suspension, nanoverkapselte Suspension umfasst.

3. Zusammensetzung nach Anspruch 2, wobei die Zusammensetzung eine wässrige Dispersion ist.

4. Zusammensetzung nach Anspruch 3, umfassend eine organische oder anorganische polyprotische Säure in Form eines Salzes mit einem Metallkation sowie Mischungen davon mit einer Pufferfunktion.

5. Zusammensetzung nach Anspruch 4, wobei die organische polyprotische Säure in Form eines Salzes mit einem Metallkation Calciumcitrat im Bereich von 100 g/L bis 150 g/L ist.

6. Zusammensetzung nach den Ansprüchen 2 bis 5, wobei die Menge der Orthokieselsäure im Bereich von 2,2 g/L bis 2,4 g/L liegt.

7. Zusammensetzung nach den Ansprüchen 2 bis 5, wobei die Menge an Vitamin D3 im Bereich von 1,7 mg/L bis 2,0 mg/L liegt, und die Menge an Vitamin K2 im Bereich von 1,3 mg/L bis 3 mg/L liegt.

8. Zusammensetzung nach den Ansprüchen 2 bis 5, **dadurch gekennzeichnet, dass** die Zusammensetzung in einer Tagesdosis im Bereich von 15 mL bis 20 mL verabreicht wird.

9. Zusammensetzung nach Anspruch 1, **dadurch gekennzeichnet, dass** die Zusammensetzung eine feste orale Form ausgewählt aus einer Gruppe ist, die Tablette, Kapsel, Sachet, Granulat, Pulver einschließt.

10. Zusammensetzung nach Anspruch 9, wobei die Äquivalente an Orthokieselsäure im Bereich von 10 mg/feste orale Form bis 300 mg/feste orale Form liegen.

11. Zusammensetzung nach den Ansprüchen 9 und 10, wobei die Menge an Vitamin D3 im Bereich von 2 IE/feste orale Form bis 2000 IE/feste orale Form liegt, und die Menge an Vitamin K2 im Bereich von 10 µg/feste orale Form bis 200 µg/feste orale Form liegt.

12. Zusammensetzung nach einem der vorhergehenden Ansprüche zur Verwendung in der Prävention oder Behandlung von Osteoporose und mit Knochenbrüchigkeit zusammenhängender Dysfunktion.

## Revendications

1. Composition comprenant une quantité thérapeutiquement efficace d'acide orthosilicique, de vitamine D3 et de vitamine K2,
**caractérisée en ce que** ladite composition fournit un effet synergique.

2. Composition selon la revendication 1, **caractérisée en ce que** ladite composition est une composition liquide destinée à la voie orale, choisie dans un groupe comprenant une dispersion aqueuse, une émulsion H/E, une émulsion E/H, un hydrogel, une suspension microencapsulée, une suspension nanoencapsulée.

3. Composition selon la revendication 2, ladite composition étant une dispersion aqueuse.

4. Composition selon la revendication 3, comprenant un acide polyprotique organique ou inorganique sous la forme d'un sel avec un cation métallique, et leurs mélanges, présentant une fonction de tampon.

5. Composition selon la revendication 4, ledit acide polyprotique organique sous la forme d'un sel avec un cation métallique étant le citrate de calcium se situant dans une plage de 100 g/l à 150 g/l.

6. Composition selon les revendications 2 à 5, la quantité dudit acide orthosilicique se situant dans une plage de 2,2 g/l à 2,4 g/l.

7. Composition selon les revendications 2 à 5, la quantité de vitamine D3 se situant dans la plage de 1,7 mg/l à 2,0 mg/l et la quantité de vitamine K2 se situant dans la plage de 1,3 mg/l à 3 mg/l.

8. Composition selon les revendications 2 à 5, **caractérisée en ce que** ladite composition est administrée en une dose quotidienne se situant dans la plage de 15 ml à 20 ml.

9. Composition selon la revendication 1, **caractérisée en ce que** ladite composition est une forme orale solide choisie dans un groupe comprenant un comprimé, une capsule, un sachet, des granules, une poudre.

10. Composition selon la revendication 9, les équivalents d'acide orthosilicique se situant dans la plage de 10 mg/forme orale solide à 300 mg/forme orale solide.

11. Composition selon les revendications 9 et 10, la quantité de vitamine D3 se situant dans la plage de 2 Ul/forme orale solide à 2000 UI/forme orale solide et la quantité de vitamine K2 se situant dans la plage de 10 µg/forme orale solide à 200 µg/forme orale solide

12. Composition selon l'une quelconque des revendications précédentes destinée à une utilisation dans la prévention ou le traitement de l'ostéoporose et d'un dysfonctionnement associé à une fragilité osseuse.
